Europäisches Patentamt

European Patent Office

Office européen des brevets

(19)

(11) Veröffentlichungsnummer : **0 514 724 B1**

(12) **EUROPÄISCHE PATENTSCHRIFT**

(45) Veröffentlichungstag der Patentschrift :
06.07.94 Patentblatt 94/27

(51) Int. Cl.$^5$ : **B01J 20/26**, A61L 15/00

(21) Anmeldenummer : **92107810.1**

(22) Anmeldetag : **08.05.92**

(54) **Pulverförmiges Absorptionsmittel für wässrige Flüssigkeiten auf Basis eines wasserquellbaren Polymeren.**

(30) Priorität : **18.05.91 DE 4116428**

(43) Veröffentlichungstag der Anmeldung :
**25.11.92 Patentblatt 92/48**

(45) Bekanntmachung des Hinweises auf die
Patenterteilung :
**06.07.94 Patentblatt 94/27**

(84) Benannte Vertragsstaaten :
**AT BE CH DE DK ES FR GB GR IT LI LU MC NL
PT SE**

(56) Entgegenhaltungen :
EP-A- 0 083 022
EP-A- 0 420 515
EP-A- 0 450 924
FR-A- 2 525 121

(73) Patentinhaber : **Chemische Fabrik
Stockhausen GmbH
Bäkerpfad 25
D-47805 Krefeld (DE)**

(72) Erfinder : **Brehm, Helmut
Dachsstrasse 22
W-4150 Krefeld (DE)**
Erfinder : **Mertens, Richard
Dahlerdyk 116a
W-4150 Krefeld (DE)**

(74) Vertreter : **Klöpsch, Gerald, Dr.-Ing.
Patentanwalt
An Gross St. Martin 6
D-50667 Köln (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 514 724 B1

## Beschreibung

Die Erfindung betrifft pulverförmige Absorptionsmittel für wässrige Flüssigkeiten auf Basis eines wasserquellbaren Polymeren, die beim Kontakt mit Wasser oder wäßrigen Lösungen, wie z.B. Körperflüssigkeiten, nicht verbacken, d.h. nicht durch Aufbau einer Barriere aus gequollenen Gelpartikeln die Absorption weiterer Flüssigkeit behindern und die ferner unter Belastung, wie sie bei Verwendung der Polymeren in Windeln und Inkontinenzartikeln gegeben ist, ihre Quellfähigkeit weitgehend beibehalten.

Die Herstellung wasserquellbarer Polymerer ist bekannt. Ein typisches Verfahren zur Herstellung wasserquellbarer Polymerpartikel durch inverse Suspensionspolymerisation einer wäßrigen Lösung aus Acrylsäure und Alkali- bzw. Ammoniumacrylat in einem Kohlenwasserstoff und Vernetzung des Polymeren wird in der US-PS 4,698,404 beschrieben.

Die Euröpäische Patentanmeldung A1 0 205 674 beschreibt die Lösungspolymerisation im wesentlichen säuregruppentragender Monomerer in Gegenwart eines vernetzend wirkenden Mittels. Nach der Neutralisation der Säuregruppen des Polymeren zu mindestens 27 % und Trocknung des Polymergels wird ein wasserquellbares Polymeres von hoher Gelstärke und mit geringen Mengen an extrahierbaren Anteilen gewonnen.

Die Europäische Patentanmeldung A2 0 312 952 beschreibt die Polymerisation von Carboxylgruppen tragenden Monomeren, die zu mindestens 20 % neutralisiert sind, in Gegenwart dispergierter Vernetzer.

Die kontinuierliche Copolymerisation einer Acrylsäure enthaltenden Monomerlösung, wobei die Acrylsäure zumindest zu 20 % in neutralisierter Form vorliegt, wird in der Europäischen Patentanmeldung A2 0 280 541 beschrieben.

Um die anwendungstechnischen Eigenschaften der wasserquellbaren Polymeren, ausgewählt aus der Gruppe bestehend aus einem vernetzten Hydrolysat eines Acrylamidcopolymeren, einem Hydrolysat eines vernetzten Acrylnitrilcopolymeren, einem vernetzten, teilweise neutralisierten Acrylsäurepolymeren zu verbessern, bzw. an ihre besondere Aufgabe anzupassen, werden sie vielfach nachbehandelt.

So beschreiben die Europäischen Patentschriften 0 009 977 und 0 001 706 die Oberflächenbeschichtung teilchenförmiger, wasserquellbarer Polymerer mit hydrophoben Verbindungen, bzw. mit Polyethern, mit dem Ziel, die Dispergierbarkeit in Blut zu verbessern.

Die US-PS, 4,587,308 beschreibt die Nachvernetzung wasserquellbarer Polymerer, dispergiert in angequollener Form, mit Diglycidethern, polyvalenten Metallsalzen oder Halogenepoxiverbindungen.

Die DE-OS 34 29 379 beschreibt ein wasserabsorbierendes Harz, erhältlich durch Imprägnation eines wasserquellbaren Harzes mit einem hydrophilen, reaktiven, olefinisch ungesättigten Monomeren oder Polymeren daraus und einem Vernetzungsmittel.

Die US-PS 4,666,975 beschreibt die Nachvernetzung wasserquellbarer Polymerer in Suspension durch Umsetzung mit Diglycidethern.

Die GB 21 19 384 A beansprucht die Nachvernetzung in der Partikeloberfläche wasserquellbarer Polymerer, deren Carboxylgruppen zu 50 bis 99 Mol% in neutralisierter Form vorliegen, mit mehrfunktionellen Verbindungen wie Polyolen oder Polyaminen.

In der DE-OS 35 23 617 und der EP-A 83 022) wird die Nachvernetzung in der Polymeroberfläche mit Lösungen von Polyolen bzw. von Diglycidylverbindungen durchgeführt. Die Nachbehandlung erfolgt an Polymeren, deren Carboxylgruppen zu 50 bis 99 Mol% neutralisiert vorliegen in Gegenwart von Wasser und einem hydrophilen organischen Lösungsmittel.

Bei all diesen Verfahren wird angestrebt, den Flüssigkeitstransport in einer Schicht aus Polymerpartikeln zu verbessern oder ein Polymer bereitzustellen, das nach dem Quellvorgang bei Ausübung eines Druckes, z. B. durch den Kinderkörper auf das gequollene Polymer in einer Windel, die gespeicherte Flüssigkeit nicht wieder abgibt.

Die Aufgabe ist aber, nicht nur den Flüssigkeitstransport in einer Schicht aus wasserquellbaren Polymerpartikeln oder die Fähigkeit des Polymeren, Flüssigkeit unter der nachfolgenden Einwirkung eines Druckes zurückzuhalten und zu verbessern, nachdem das Polymer frei, d.h. ohne Belastung quellen konnte, sondern ein wasserquellbares Polymer bereitzustellen, das Flüssigkeit auch gegen eine ausgeübte Belastung aufnimmt. Diese spezielle Absorptionseigenschaft wird in der EP 0 339 461 als Aufnahme unter Druck bezeichnet.

Es wurde nun gefunden, daß wasserquellbare, Carboxylgruppen tragende Polymere, die zu 50 bis 99 Mol% in neutralisierter Form vorliegen, mit hoher Quellkapazität unter Belastung gewonnen werden können, wenn man wasserquellbare Polymere mit geringer Quellkapazität unter Belastung mit N-(Hydroxialkyl)β-(Meth)-alaninestern und/oder deren Polykondensationsprodukten behandelt und erwärmt.

Weiter wurde gefunden, daß die Nachbehandlung bei erhöhten Temperaturen von Carboxylgruppen tragenden Polymeren, die zu mehr als 80 Mol% in neutralisierter Form vorliegen, mit Polyolen oder Aminoalkoholen nicht oder nur unter nicht praktikablen Bedingungen zu Produkten mit verbesserten Eigenschaften führt, im Gegensatz dazu aber durch die Behandlung dieser zu mehr als 80 Mol% neutralisierten Polymeren mit N-(Hydroxialkyl)β-(Meth)alaninestern und/oder deren Polykondensationsprodukten bei erhöhten Temperaturen eine Erhöhung der Quellkapazität unter Belastung erreicht werden kann.

Als weiterer technischer Vorteil ergibt sich bei der Nachbehandlung wasserquellbarer Polymerer mit N-(Hydroxialkyl)β-(Meth)alaninestern und/oder den daraus entstehenden Polyamiden bzw. Polyamidethern, daß gegenüber Polyolen oder Alkanolaminen, entsprechend den Verfahrensweisen nach GB 21 19 384 und DE-OS 35 33 617, niedrigere Nachbehandlungstemperaturen bei molar gleicher Einsatzmenge an Nachbehandlungsmittel bzw. kürzere Nachbehandlungszeiten bei gleicher Temperatur erforderlich sind, um eine Verbesserung der Produkteigenschaften zu erreichen.

Die Herstellung der N-(Hydroxialkyl)β-(Meth-)Alaninester und ihre Fähigkeit, bereits bei Raumtemperatur Polyamide bzw. Polyamidether zu bilden, wird in Journal of Polymer Science: Part A-1, Vol. 6, 1195-1207 (1968) und Vol. 7, 889-898 (1969) ausführlich beschrieben.

Ihre Herstellung erfolgt durch Michael-Addition von Alkanolen oder Alkanolaminen an (Meth)Acrylsäureester.

Die Menge der erfindungsgemäß eingesetzten N-(Hydroxialkyl)β-(Meth)alaninester und/oder ihrer Polykondensationsprodukte beträgt 0,001 bis 5 Gew.-Teile bezogen auf 100 Teile wasserquellbares Polymer. Werden mehr als 5 Gew.-Teile verwendet, so nimmt die Quellkapazität (Retention) der Polymeren zu stark ab. Unterhalb einer Einsatzmenge von 0,001 Gew.-Teile wird die Quellkapazität unter Belastung nicht verbessert.

Eingesetzt werden die erfindungsgemäßen Nachbehandlungsmittel in wäßriger Lösung. Das Wasser dient dazu, die in Alkohol unlöslichen, festen Polyamide der N-(hydroxialkyl)β(meth)alaninester zu lösen und dient gleichzeitig als Vehikel für die Nachbehandlungsmittel in die wasserquellbaren Polymerpartikel. Die Menge an Wasser beträgt 0,1 bis 5 Gew.-Teile auf 100 Gew.-Teile wasserquellbares Polymer. Da die nicht nachbehandelten wasserquellbaren Polymeren bei Kontakt mit den wäßrigen Lösungen des Nachbehandlungsmittels verbacken würden, werden noch zusätzlich wasserlösliche, organische Verdünnungsmittel in Mengen von 0,2 bis 10 Gew.-Teile pro 100 Gew.-Teile wasserquellbares Polymer mitverwendet. Als wasserlösliche organische Verdünnungsmittel eingesetzt werden:

a) solche, die bei der Erwärmung der Mischung aus der Lösung des Nachbehandlungsmittels und dem wasserquellbaren Polymeren verdampfen, wie z.B. monofunktionelle Alkohole der C-Zahl 1-4, Aceton, Glykolether, Glykolester und/oder

b) solche, die bei der Erwärmung nicht verdampfen und auf bzw. im Polymerpartikel verbleiben, wie z.B. Butyrolacton, Butyrolactam, Milchsäure, Diacetin.

Die unter a) aufgeführten Verdünnungsmittel erfordern bei der Durchführung der Nachbehandlung der wasserquellbaren Polymeren gewisse technische Maßnahmen, da sie brennbar sind, explosionsfähige Gemische bilden und nicht in die Atmosphäre gelangen dürfen.

Die unter b) aufgeführten Verdünnungsmittel verbleiben auf dem Polymeren und erfordern keine besonderen Schutzmaßnahmen. In höheren Mengen bei der Nachbehandlung eingesetzt, können sie aber einen negativen Einfluß auf die Quellkapazität der nachbehandelten Polymeren haben.

Die Menge des zugegebenen wasserlöslichen organischen Verdünnungsmittels ist abhängig von der Menge an eingesetztem Wasser. Sie soll so bemessen sein, daß ein handhabbares Produkt, das keine verklebten Partikel enthält, nach dem Vermischen mit dem wasserquellbaren Polymeren vorliegt.

Die Lösung des Behandlungsmittels wird mit dem pulverförmigen, wasserquellbaren, Carboxylgruppen tragenden Polymer vermischt und anschließend erwärmt. Das pulverförmige Polymer kann von kugeliger Gestalt sein, erhältlich durch Polymerisation emulgierter, wäßriger Monomerlösungen oder eine unregelmäßige Form besitzen, erhältlich durch Lösungspolymerisation, beispielsweise einer 30%igen Monomerlösung, die 50 bis 99 Mol% Alkaliacrylat, 50 bis 1 Mol% Acrylsäure und 0,01 bis 0,5 Gew% eines vernetzend wirkenden Mittels enthält. Die Korngröße des zu behandelnden wasserquellbaren Polymeren beträgt 0,05 bis 1 mm, bevorzugt 0,1 bis 0,8 mm.

Die Mischung aus der Lösung des Behandlungsmittels und des wasserquellbaren Polymeren wird im Labor mit einfachen Mischern, wie sie im Haushalt verwendet werden, hergestellt. In der Technik verwendet man kontinuierlich oder diskontinuierlich arbeitende Schaufel- oder Paddelmischer. Die Lösung des Behandlungsmittels wird dabei gleichmäßig zur Pulvervorlage oder in den Pulverstrom dosiert.

Die Mischung aus gelöstem, verdünntem Behandlungsmittel und wasserquellbarem Polymer wird anschließend auf 100 bis 250°C, bevorzugt 150 bis 210°C, erwärmt. Die Erwärmungstemperatur und -zeit ist abhängig von der Menge an Behandlungsmittel, wobei die Behandlungszeit in der Regel zwischen wenigstens 5 Min. und einer Stunde liegt.

Vorteilhaft werden die Menge an Behandlungsmittel, die Zugabeweise und die Behandlungstemperatur so aufeinander abgestimmt, daß die Behandlungszeit eine kontinuierliche Durchführung des Verfahrens erlaubt, d.h., daß die erforderliche Aufheizzeit zu nicht zu großen Verweilzeiten im Trockner und anschließenden Kühler führt. Als Trockner eignet sich z.B. der Trockenschrank oder der Tellertrockner.

Gewonnen wird ein wasserquellbares Polymer, das nach dem Quellvorgang einen trockenen Eindruck vermittelt, das Vordringen von Flüssigkeit durch Aufbau einer Barriere nicht behindert und bei hoher Quellkapazität (Retention) gegen eine äußere Belastung anquillt. Daher ist das erfindungsgemäße Polymer besonders zum Einbetten zwischen Papier, nicht gewebten Textilien und in Windelkonstruktionen geeignet.

Das erhaltene nachbehandelte Polymer wird wie folgt geprüft:

1.) Flüssigkeitsaufnahme

Gemessen wird die aufgenommene Menge an Flüssigkeit pro Gramm wasserquellbares Polymeres. Eine gewogene Menge Polymeres wird in einen Teebeutel eingeschlossen und in eine 0,9%ige Kochsalzlösung getaucht. Ermittelt wird die Aufnahmemenge nach 10 Min. nach Abschleudern des Teebeutels bei 1400 Upm und 5 Min. in einer Zentrifuge von 23 cm Durchmesser, wobei die Menge Flüssigkeit, die das Material des Teebeutels aufgenommen hat, abgezogen wird.

$$\text{Aufnahme-menge} \left(\frac{g}{g}\right) = \frac{\text{Gewicht nach der Adsorption (g)} - \text{Gewicht des Teebeutels (g)}}{\text{Gewicht des Polymeren (g)}}$$

(Retention)

2) Flüssigkeitsaufnahme unter Belastung

Die Aufnahme unter Belastung (AUB) wird nach der in der EP 0 339 461, Seite 7, beschriebenen Methode bestimmt: In ein zylindrisches Gefäß mit Siebboden gibt man die Einwaage an Superabsorber und belastet das Pulver mit einem Stempel, der einen Druck von 20 g/cm$^2$ ausübt. Der Zylinder wird anschließend auf einen Demand-Absorbency-Tester (DAT) gestellt, wo man den Superbabsorber eine Stunde lang 0,9%ige NaCl-Lösung saugen läßt.

3) Schermodul

Der Schermodul wird mit einem Carri-Med-Stress-Rheometer mit einer Platte-Platte-Konfiguration gemessen. Zur Bestimmung des Schermoduls läßt man 1 g wasserabsorbierendes Harz in 28 g = 0,9%iger NaCl-Lösung für 1 Std. quellen und mißt anschließend an diesem gequollenen Gel den Schermodul in Abhängigkeit von der Frequenz (0,1 - 10 Hz). Der Wert bei 10 Hz wird als Speichermodul G′ in N/m$^2$ angegeben.

Die Erfindung wird anhand der nachfolgenden Beispiele näher erläutert:

1.) Herstellung der erfindungsgemäß eingesetzten Behandlungsmittel für die wasserquellbaren Polymeren (N-(Hydroxialkyl)β-(Meth)-alaninester und deren Polykondensationsprodukte:

a) Behandlungsmittel A

10 g Ethylacrylat und 10 g Ethanol werden in einen Kolben eingewogen und unter Rühren mit 6,15 g Ethanolamin versetzt. Die sich erwärmende Lösung läßt man 15 Std. zur Abreaktion stehen.

b) Behandlungsmittel B

10 g Methylmethacrylat und 6,15 g Ethanolamin werden bei 40°C unter Rühren 15 Std. lang zur Reaktion gebracht.

c) Behandlungsmittel C

13 g 2-Hydroxipropylacrylat und 10 g Ethanol werden in einem Kolben bei Raumtemperatur mit 6,2 g Ethanolamin verrührt. Die Lösung wird 18 Std. lang zur Abreaktion stehengelassen.

d) Behandlungsmittel D

Behandlungsmittel A, eine klare Lösung, läßt man 14 Tage bei Raumtemperatur stehen. Während dieser Zeit bildet sich ein feiner weißer Niederschlag, der in Alkohol oder Wasser löslich ist. Zur Behandlung der Polymeren wird die homogenisierte Suspension verwendet.

e) Behandlungsmittel E

100 g Ethylacrylat und 100 g Ethanol werden in einem Rührkolben mit 61 g Ethanolamin vermischt und die klare Lösung nach 18 Std. in einem Rotationsverdampfer bei einer Badtemperatur von 80°C und einem Druck von 20 mbar von flüchtigen Anteilen befreit. Gewonnen wird ein bei Raumtemperatur leicht trübes, hochviskoses Öl, das beim Stehen wachsartig wird.

f) Behandlungsmittel F

61 g Monoethanolamin in 200 ml Methanol werden in einem Rührkolben mit 100 g Ethylacrylat versetzt und 8 Std. lang gerührt. In den Rührkolben gibt man 800 ml Methanol und 14 g 25%ige Kaliummethylatlösung und rührt 80 Std. lang. Der entstandene weiße Niederschlag wird abfiltriert, in 500 ml Methanol aufgerührt und erneut abfiltriert. Getrocknet wird der Filterkuchen bei 50°C und 25 mbar.

Gewonnen werden 73,2 g weißes Pulver, das zwischen 195 und 203°C schmilzt.

g) Behandlungsmittel G

In einem Rührkolben werden 1000 ml Tetrahydrofuran vorgelegt und unter Rühren mit 14 g 25%iger Kaliummethylatlösung, 62 g Monoethanolamin und 100 g Ethylacrylat versetzt. Aus der anfangs klaren Lösung scheidet sich nach Eintrübung der Flüssigkeit ein weiches Polykondensat am Boden des Reaktors ab. Die überstehende Lösung wird abgegossen und das viskose Polykondensat bei 25 mbar und 50°C von anhaftenden flüchtigen Anteilen befreit.

Ges.-Amin: 2,8 mmol/g.

2.) Behandlung der wasserquellbaren beim Polymeren gemäß Erfindung:

Beispiel 1 und 2 und Vergleichsbeispiele 1 bis 3

Ein wasserquellbares Polymeres der Korngröße 0,2 bis 0,8 mm, erhalten durch Polymerisation einer 29%igen Monomerenlösung aus 90 Mol% Natriumacrylat und 10 Mol% Acrylsäure in Gegenwart von 0,25 Gew.% Trimethylol-propan-triacrylat (bezogen auf Acrylsäure) mit einem Aufnahmevermögen (Retention) für 0,9%ige NaCl-Lösung von 40,0 g/g und ein Aufnahmevermögen unter Belastung (AUB) von 6,9 g/g.

100 g des wasserquellbaren Polymeren werden in einem 500 ml Kunststoffbecher vorgelegt und unter kräftigem Rühren mit einem einarmigen Haushaltsmischer mit den in der Tab. 1 angegebenen Mengen Nachbehandlungsmittel, Wasser und organ. Verdünnungsmittel in ca. 3 Min. vermischt. Die Mischung wird in einer Glasschale in einer 5 bis 10 mm starken Schicht ausgebreitet und in einem Umlufttrockenschrank erwärmt. Tocknungszeiten und Temperaturen sowie die Prüfungsergebnisse sind in Tab. 1 aufgelistet. In den Vergleichsbeispielen 1 - 3 wurden als Behandlungsmittel Ethanolamin und Glycerin verwendet.

Tabelle 1

| Beisp. Nr. | Wasser (g) | Verd.-mittel (g) | Behandl.-mittel (g) | zeit (min) | temp. (°C) | AUB (g/g) | Retention (g/g) |
|---|---|---|---|---|---|---|---|
| 1 | 2,0 | Methanol 2,8 | E 1,3 | 10 | 190 | 24,0 | 32,6 |
| 2 | 2,0 | Methanol 2,4 | A 1,0 | 20 | 190 | 30,9 | 33,2 |

Vergleichsbeispiel

| Beisp. Nr. | Wasser (g) | Verd.-mittel (g) | Behandl.-mittel (g) | zeit (min) | temp. (°C) | AUB (g/g) | Retention (g/g) |
|---|---|---|---|---|---|---|---|
| 1 | 2,0 | Methanol 2,8 | Ethanolamin 0,5 | 10 | 190 | 6,9 | 39,6 |
| " | " | " | " | 20 | 190 | 7,5 | 40,7 |
| 2 | 2,0 | Methanol 2,8 | Glycerin 0,5 | 20 | 190 | 8,2 | 38,1 |
| " | " | " | " | 60 | 190 | 8,6 | 39,6 |
| 3 | 2,0 | Methanol 2,8 | 1,0 | 30 | 190 | 13,8 | 37,9 |

Beispiel 3 bis 5

Ein Polymeres der Korngröße 0,15 - 0,8 mm, gewonnen wie in Beispiel 1 durch Lösungspolymerisation, mit der Ausnahme, daß die Monomermischung zu 70 Mol% aus Natriumacrylat und zu 30 Mol% aus Acrylsäure besteht. Das Polymere hat eine Retention von 39,5 g/g und eine AUB von 7,6 g/g.

100 g dieses pulverförmigen Polymeren werden wie in Beispiel 1 mit Lösungen des Behandlungsmittels F vermischt und erwärmt. Die Behandlungsbedingungen und Prüfergebnisse sind in Tabelle 2 aufgelistet.

Tabelle 2

| Beisp. Nr. | Wasser (g) | Verd.- mittel (g) | Behandl.- mittel (g) | zeit (min) | temp. (°C) | AUB (g/g) | Reten- tion (g/g) |
|---|---|---|---|---|---|---|---|
| | | Methanol | | | | | |
| 3 | 2,0 | 2,8 | 0,30 | 15 | 180 | 25,8 | 37,3 |
| | | Methanol | | | | | |
| 4 | 2,0 | 2,8 | 0,15 | 15 | 180 | 28,6 | 37,6 |
| | | Ethanol | | | | | |
| 5 | 2,0 | 2,8 | 0,05 | 20 | 190 | 32,6 | 38,1 |

Beispiel 6

100 g des in Beispiel 3 eingesetzten wasserquellbaren Polymeren werden wie in Beispiel 1 mit einer Lösung aus 0,1 g Behandlungsmittel E, 2 g Wasser und 1,0 g Pyrrolidon-2 vermischt und erwärmt.

| Behandlungs- temp. (°C) | zeit (min) | AUB (g/g) | Retention (g/g) |
|---|---|---|---|
| 190 | 10 | 26,2 | 40,4 |
| 190 | 15 | 30,4 | 36,2 |
| 190 | 20 | 29,2 | 34,9 |

Beispiel 7 bis 12 und Vergleichsbeispiel 4

Durchgeführt wird die Mischung mit 100 g Polymeren wie in Beispiel 3 unter den in Tabelle 3 aufgeführten Bedingungen und mit den dort angegebenen Nachbehandlungsmitteln, -temperaturen und -zeiten.

## Tabelle 3

| Beisp. Nr. | Wasser (g) | Lösungs- mittel (g) | Behandlungs- mittel (g) | temp. (°C) | zeit (min) | AUB (g/g) | Reten- tion (g/g) |
|---|---|---|---|---|---|---|---|
| 7 | 2,0 | - | A 8,58 | 150 | 10 | 27,9 | 39,0 |
| 8 | 2,0 | Methanol 2 | A 2,11 | 180 | 10 | 30,3 | 34,1 |
| 9 | 2,0 | Methanol 2 | A 1,05 | 180 | 14 | 31,1 | 36,0 |
| 10 | 2,0 | Methanol 2 | A 0,52 | 180 | 20 | 28,7 | 36,1 |
| 11 | 2,0 | Methanol 2 | B 5,27 | 150 | 60 | 31,3 | 35,4 |
| 12 | 2,0 | Ethanol 2 | C 2,4 | 150 | 30 | 27,3 | 33,6 |

Vergleichsbeispiel

| | Wasser (g) | Ethanol (g) | Ethanolamin (g) | temp. (°C) | zeit (min) | AUB (g/g) | |
|---|---|---|---|---|---|---|---|
| 4 | 2,0 | 3,85 | 0,5 | 150 | 30 | 9,0 | |
| | 2,0 | 3,85 | 0,5 | 150 | 60 | 10,5 | |

Beispiel 13

100 g des in Beispiel 3 eingesetzten wasserquellbaren Polymeren (G' = 1800 N/m²) werden mit 5,05 g einer Lösung aus 1,05 g der homogenisierten Suspension des Nachbehandlungsmittels D, 2,0 g Wasser und 2,0 g Methanol vermischt und im Umlufttrockenschrank erwärmt.

| Behandlungs-zeit (min) | temp. (°C) | AUB (g/g) | Retention (g/g) | G' (N/m²) |
|---|---|---|---|---|
| 10 | 180 | 26,8 | 36,7 | 2350 |

## Beispiel 14 bis 16

100 g einer vernetzten Polyacrylsäure, deren Carboxylgruppen zu 60 Mol% in neutralisierter Form vorliegen, der Korngröße 0,2 bis 0,85 mm, mit der Bezeichnung FAVOR 922, ein Produkt der Chemischen Fabrik Stockhausen GmbH, Krefeld, werden mit einer Lösung des Nachbehandlungsmittels A in 2,0 g Wasser und 2,0 g Methanol wie in Beispiel 1 vermischt und in einem Umlufttrockenschrank erwärmt.

| Beispiel | Behandlungs-mittel (g) | temp. (°C) | zeit (min) | AUB (g/g) | Retention (g/g) |
|---|---|---|---|---|---|
| FAVOR | - | - | - | 9,3 | 41,9 |
| 14 | 2,1 | 180 | 10 | 25,1 | 30,2 |
| 15 | 4,2 | 160 | 10 | 26,4 | 34,5 |
| 16 | 4,2 | 150 | 15 | 28,2 | 34,1 |

## Beispiel 17

1500 g wasserquellbares Polymer der Korngröße 0,15 bis 0,85 mm, hergestellt durch Polymerisation einer 29%igen Lösung von 70 Mol% Natriumacrylat, 30 Mol% Acrylsäure und 0,17 Gew.% Trimethylol-propan-triacrylat (bezogen auf Acrylsäure) mit einer Retention von 44,8 g/g und einer AUB von 7,2 g/g, werden in einem Draismischer vorgelegt. In das bewegte Pulverbett wird eine Lösung aus 1,5 g Behandlungsmittel E, 30 g Wasser und 15 g Pyrrolidon-2 getropft. Die erhaltene Mischung wird in einem auf 210°C vorgeheizten Freifallmischer erwärmt. Die Erwärmungszeiten, -temperaturen und Prüfungsergebnisse sind in Tabelle 4 aufgelistet.

## Tabelle 4

| Zeit (min) | Temperatur (°C) | | AUB (g/g) | Retention (g/g) |
|---|---|---|---|---|
| 10 | 146 | } Aufheizphase | | |
| 20 | 177 | | | |
| 25 | 183 | | – | – |
| 30 | 183 | | 30,2 | 40,4 |
| 40 | 185 | | 29,6 | 38,6 |
| 50 | 186 | | 28,7 | 37,8 |

### Beispiel 18

100 g des in Beispiel 3 eingesetzten wasserquellbaren Polymeren wird mit einer Lösung aus 0,3 g Behandlungsmittel G in 2,0 g Wasser und 2,8 g Methanol vermischt, in einer Glasschale ausgebreitet und für 15 Min. in einem Trockenschrank von 190°C gelagert. Das auf Raumtemperatur abgekühlte Produkt zeigt kein Blocken beim Quellvorgang, eine AUB von 33,5 g/g und eine Retention von 37,7 g/g.

### Beispiel 19

100 g des in Beispiel 3 eingesetzten wasserquellbaren Polymeren wird mit einer Lösung aus 0,1 g Behandlungsmittel E in 2,0 g Wasser und 1,0 g Diacetin vermischt. Zur Trocknung wird das pulverförmige Produkt für 15 Min. in einen auf 190°C vorgeheizten Trockenschrank gegeben. Nach dem Abkühlen wird eine AUB von 29,9 g/g gemessen.

### Beispiel 20

Eingesetzt wird ein vernetztes, wasserquellbares Polymer der Korngröße 0,15 - 0,80 mm und der Zusammensetzung entsprechend Beispiel 3. Das Polymere hat eine Retention von 35,5 g/g, eine AUB von 9,4 g/g und eine Speichermodul von 2600 N/m$^2$.

Eine Mischung aus 1,5 g Behandlungsmittel, hergestellt wie Behandlungsmittel A ohne Verwendung von Ethanol als Verdünnungsmittel, 30 g Wasser und 30 g Diacetin, wird wie in Beispiel 17 mit 1500 g des pulverförmigen Polymeren vermischt und erwärmt. Nach einer Behandlungszeit von 20 Min. bei 183 bis 185°C zeigt das Polymere folgende Kenndaten:

Retention = 30,7 g/g
AUB = 31,1 g/g
G′ = 5300 N/m$^2$

### Beispiel 21

100 g einer vernetzten Polyacrylsäure, deren Carboxylgruppen zu 70 Mol% in neutralisierter Form vorliegen, mit einer Retention von 47,5 g/g und einer AUB von 8,4 g/g, werden mit 4,1 g der Behandlungsmischung aus Beispiel 20 behandelt und 10 Min. in einem auf 240°C vorgeheizten Trockenschrank in 3 mm Schichtstärke gelagert. Das gewonnene Polymere hat eine Retention von 35,5 g/g und eine AUB von 24,4 g/g.

**Patentansprüche**

**Patentansprüche für folgende Vertragsstaaten : AT, BE, CH, DE, DK, FR, GB, IT, LU, LI, MC, NL, PT, SE**

1. Pulverförmiges Absorptionsmittel für wässrige Flüssigkeiten auf Basis eines wasserquellbaren, pulverförmigen Polymers, erhältlich durch Herstellen und Erwärmen einer Mischung, bestehend aus 100 Gew.-Tl wenigstens eines wasserquellbaren carboxylgruppenhaltigen Polymeren, das mindestens 0,2 Äquivalente Carboxylgruppen pro 100 Gew.-Tl enthält, die zu 50 bis 99 Mol% in neutralisierter Form vorliegen,
   a) 0,001 bis 5 Gew.-Tl, bevorzugt 0,05 bis 2 Gew.-Tl, N-(Hydroxialkyl)$\beta$-(Meth)- Alaninestern und/oder
   b) 0,001 bis 5 Gew.-Tl, bevorzugt 0,05 bis 2 Gew.-Tl, Polykondensationsprodukten von a) in
   c) 0,1 bis 5 Gew.-Tl Wasser und
   $d^1$) 0,2 bis 10 Gew.-Tl eines mit Wasser mischbaren organischen Verdünnungsmittels, das beim Erwärmen der Mischung verdampft und/oder
   $d^2$) 0,2 bis 10 Gew.-Tl eines mit Wasser mischbaren organischen Verdünnungsmittels, das beim Erwärmen der Mischung nicht verdampft.

2. Pulverförmiges Absorptionsmittel nach Anspruch 1, dadurch erhalten, daß man die Mischung nach Anspruch 1 auf 100 bis 250°C, bevorzugt auf 150 bis 210°C, erwärmt.

3. Pulverförmiges Absorptionsmittel nach Anspruch 1 und 2, dadurch erhalten, daß man am Stickstoffatom durch Hydroxi-$C_1$-$C_6$-alkylgruppen substituierte $\beta$-(Meth)-Alaninester verwendet.

4. Pulverförmiges Absorptionsmittel nach einem der Ansprüche 1 bis 3, dadurch erhalten, daß man N-(Hydroxialkyl)-substituierte $\beta$-(Meth)Alanin-$C_1$-$C_6$-alkylester oder -$C_1$-$C_6$-hydroxialkyl-ester verwendet.

5. Pulverförmiges Absorptionsmittel nach einem der Ansprüche 1 bis 4, dadurch erhalten, daß man wasserlösliche Polyamide von N-(hydroxialkyl)$\beta$-(Meth-)Alaninestern verwendet.

6. Pulverförmiges Absorptionsmittel nach einem der Ansprüche 1 bis 4, dadurch erhalten, daß man wasserlösliche Polyamidether von N-(hydroxialkyl)$\beta$-(Meth-)Alaninestern verwendet.

7. Pulverförmiges Absorptionsmittel nach einem der Ansprüche 1 - 6, bei dem wenigstens 80 Mol% des Polymeren neutralisiert sind.

**Patentansprüche für folgenden Vertragsstaat : GR**

1. Verfahren zur Herstellung eines pulverförmigen Absorptionsmittels für wässrige Flüssigkeiten auf Basis eines wasserquellbaren, pulverförmigen Polymers, dadurch gekennzeichnet, daß diese erhältlich sind durch Herstellen und Erwärmen einer Mischung, bestehend aus 100 Gew.-Tl wenigstens eine wasserquellbaren carboxylgruppenhaltigen Polymeren, das mindestens 0,2 Äquivalente Carboxylgruppen pro 100 Gew.-Tl enthält, die zu 50 bis 99 Mol% in neutralisierter Form vorliegen
   mit
   a) 0,001 bis 5 Gew.-Tl, bevorzugt 0,05 bis 2 Gew.-Tl, N-(Hydroxialkyl)$\beta$-(Meth)- Alaninestern und/oder
   b) 0,001 bis 5 Gew.-Tl, bevorzugt 0,05 bis 2 Gew.-Tl, Polykondensationsprodukten von a) in
   c) 0,1 bis 5 Gew.-Tl Wasser und
   $d^1$) 0,2 bis 10 Gew.-Tl eines mit Wasser mischbaren organischen Verdünnungsmittels, das beim Erwärmen der Mischung verdampft und/oder
   $d^2$) 0,2 bis 10 Gew.-Tl eines mit Wasser mischbaren organischen Verdünnungsmittels, des beim Erwärmen der Mischung nicht verdampft.

2. Verfahren nach Anspruch 1, dadurch gekennzeichnet, daß man die nach Anspruch 1 erhaltene Mischung auf 100 bis 250°C, bevorzugt auf 150 bis 210°C, erwärmt.

3. Verfahren nach Anspruch 1 und 2, dadurch gekennzeichnet, daß man am Stickstoffatom durch Hydroxi-$C_1$-$C_6$-alkylgruppen substituierte $\beta$-(Meth)-Alaninester verwendet.

4. Verfahren nach einem der Ansprüche 1 bis 3, dadurch gekennzeichnet, daß man N-(Hydroxialkyl)-substituierte $\beta$-(Meth)Alanin-$C_1$-$C_6$-alkylester oder -$C_1$-$C_6$-hydroxialkylester verwendet.

5. Verfahren nach einem der Ansprüche 1 bis 4, dadurch gekennzeichnet, daß man wasserlösliche Polyamide von N-(hydroxialkyl)β(Meth-)Alaninestern verwendet.

6. Verfahren nach einem der Ansprüche 1 bis 4, dadurch gekennzeichnet, daß man wasserlösliche Polyamidether von -N-(hydroxialkyl)β-(Meth-)Alaninestern verwendet.

7. Verfahren nach einem der Ansprüche 1 - 6, dadurch gekennzeichnet, daß man wenigstens 80 Mol% des Polymeren neutralisiert.

**Patentansprüche für folgenden Vertragsstaat : ES**

1. Verfahren zur Herstellung eines pulverförmigen Absorptionsmittels für wässrige Flüssigkeiten auf Basis eines wasserquellbaren, pulverförmigen Polymers, dadurch gekennzeichnet, daβ diese erhältlich sind durch Herstellen und Erwärmen einer Mischung, bestehend aus 100 Gew.-Tl wenigstens eines wasserquellbaren carboxylgruppenhaltigen Polymeren, das mindestens C,2 Äquivalente Carboxylgruppen pro 100 Gew.-Tl enthält, die zu 50 bis 99 Mol% in neutralisierter Form vorliegen
mit
   a) 0,001 bis 5 Gew.-Tl, bevorzugt 0,05 bis 2 Gew.-Tl, N-(Hydroxialkyl)β-(Meth)- Alaninestern und/oder
   b) 0,001 bis 5 mew,-Tl, bevorzugt 0,05 bis 2 Gew.-Tl, Polykondensationsprodukten von a) in
   c) 0,1 bis 5 Gew.-Tl Wasser und
   $d^1$) 0,2 bis 10 Gew.-Tl eines mit Wasser mischbaren organischen Verdünnungsmittels, das beim Erwärmen der Mischung verdampft und/oder
   $d^2$) 0,2 bis. 10 Gew.-Tl eines mit Wasser mischbaren organischen Verdünnungsmittels, das beim Erwärmen der Mischung nicht verdampft.

2. Verfahren nach Anspruch 1, dadurch gekennzeichnet, daß man die nach Anspruch 1 erhaltene Mischung auf 100 bis 250°C, bevorzugt auf 150 bis 210°C, erwärt.

3. Verfahren nach Anspruch 1 und 2, dadurch gekennzeichnet, daß man am Stickstoffatom durch Hydroxi-$C_1$-$C_6$-alkylgruppen substituierte β-(Neth)-Alaninester verwendet.

4. Verfahren nach einem der Ansprüche 1 bis 3, dadurch gekennzeichnet, daß man N-(Hydroxialkyl)-substituierte β-(Meth)Alanin-$C_1$-$C_6$-alkylester oder -$C_1$-$C_6$-hydroxialkylester verwendet.

5. Verfahren nach einem der Ansprüche 1 bis 4, dadurch gekennzeichnet, daß man wasserlösliche Polyamide von N-(hydroxialkyl)β(Meth-)Alaninestern verwendet.

6. Verfahren nach einem der Ansprüche 1 bis 4, dadurch gekennzeichnet, daß man wasserlösliche Polyamidether von N-(hydroxialkyl)β(Meth-)Alaninestern verwendet.

7. Verfahren nach einem der Ansprüche 1 - 6, dadurch gekennzeichnet, daß man wenigstens 80 Mol% des Polymeren neutralisiert.

**Claims**

**Claims for the following Contracting States : AT, BE, CH, DE, DK, FR, GB, IT, LU, LI, MC, NL, PT, SE**

1. A powdery absorbent for aqueous liquids based on a water-swellable powdery polymer, obtainable by manufacturing and heating a mixture consisting of 100 parts by weight of at least one water-swellable carboxyl-groups-containing polymer comprising at least 0.2 equivalents of carboxyl groups per 100 parts by weight which are neutralized to the extent of 50 to 99 mol-%,
   a) 0.001 to 5 parts by weight, preferably 0.05 to 2 parts by weight of N-(hydroxyalkyl)β-(meth)-alanine esters and/or
   b) 0.001 to 5 parts by weight, preferably 0.05 to 2 parts by weight of polycondensation products of a) in
   c) 0.1 to 5 parts by weight of water and
   $d^1$) 0.2 to 10 parts by weight of a water-miscible organic diluting agent which evaporates on heating

12

EP 0 514 724 B1

the mixture and/or

d$^2$) 0.2 to 10 parts by weight of a water-miscible organic diluting agent which does not evaporate on heating the mixture.

2. The powdery absorbent according to claim 1 obtained by heating the mixture according to claim 1 to 100 to 250°C, preferably to 150 to 210°C.

3. The powdery absorbent according to claim 1 and 2 obtained by using β-(meth)-alanine esters substituted at the nitrogen atom by hydroxy-$C_1$-$C_6$-alkyl groups.

4. The powdery absorbent according to any one of claims 1 to 3 obtained by using N-(hydroxyalkyl)-substituted β-(meth)alanine-$C_1$-$C_6$-alkyl esters or -$C_1$-$C_6$-hydroxyalkyl esters.

5. The powdery absorbent according to any one of claims 1 to 4 obtained by using water-soluble polyamides of N-(hydroxyalkyl)β-(meth-)alanine esters.

6. The powdery absorbent according to any one of claims 1 to 4 obtained by using water-soluble polyamide ethers of N-(hydroxyalkyl)β-(meth-)alanine esters.

7. The powdery absorbent according to any one of claims 1 to 6 in which at least 80 mol-% of the polymer are neutralized.

**Claims for the following Contracting State : GR**

1. A process for the production of powdery absorbents for aqueous liquids, which absorbents are based on a water-swellable powdery polymer, characterized in that they are obtainable by manufacturing and heating a mixture consisting of 100 parts by weight of at least one water-swellable carboxyl-groups-containing polymer comprising at least 0.2 equivalents of carboxyl groups per 100 parts by weight which are neutralized to the extent of 50 to 99 mol-%,

with

a) 0.001 to 5 parts by weight, preferably 0.05 to 2 parts by weight of N-(hydroxyalkyl)β-(meth)-alanine esters and/or

b) 0.001 to 5 parts by weight, preferably 0.05 to 2 parts by weight of polycondensation products of a) in

c) 0.1 to 5 parts by weight of water and

d$^1$) 0.2 to 10 parts by weight of a water-miscible organic diluting agent which evaporates on heating the mixture and/or

d$^2$) 0.2 to 10 parts by weight of a water-miscible organic diluting agent which does not evaporate on heating the mixture.

2. The process according to claim 1 characterized in that the mixture obtained according to claim 1 is heated to 100 to 250°C, preferably to 150 to 210°C.

3. The process according to claim 1 and 2 characterized in that β-(meth)-alanine esters substituted at the nitrogen atom by hydroxy-$C_1$-$C_6$-alkyl groups are used.

4. The process according to any one of claims 1 to 3 characterized in that N-(hydroxyalkyl)-substituted β-(meth)alanine-$C_1$-$C_6$-alkyl esters or -$C_1$-$C_6$-hydroxyalkyl esters are used.

5. The process according to any one of claims 1 to 4 characterized in that water-soluble polyamides of N-(hydroxyalkyl)β-(meth-)alanine esters are used.

6. The process according to any one of claims 1 to 4 characterized in that water-soluble polyamide ethers of N-(hydroxyalkyl)β-(meth-)alanine esters are used.

7. The process according to any one of claims 1 to 6 characterized in that at least 80 mol-% of the polymer are neutralized.

13

**Claims for the following Contracting State : ES**

1. A process for the production of powdery absorbents for aqueous liquids, which absorbents are based on a water-swellable powdery polymer, characterized in that they are obtainable by manufacturing and heating a mixture consisting of 100 parts by weight of at least one water-swellable carboxyl-groups-containing polymer comprising at least 0.2 equivalents of carboxyl groups per 100 parts by weight which are neutralized to the extent of 50 to 99 mol-%,
with

   a) 0.001 to 5 parts by weight, preferably 0.05 to 2 parts by weight of N-(hydroxyalkyl)β-(meth)-alanine esters and/or
   b) 0.001 to 5 parts by weight, preferably 0.05 to 2 parts by weight of polycondensation products of a) in
   c) 0.1 to 5 parts by weight of water and
   $d^1$) 0.2 to 10 parts by weight of a water-miscible organic diluting agent which evaporates on heating the mixture and/or
   $d^2$) 0.2 to 10 parts by weight of a water-miscible organic diluting agent which does not evaporate on heating the mixture.

2. The process according to claim 1 characterized in that the mixture obtained according to claim 1 is heated to 100 to 250°C, preferably to 150 to 210°C.

3. The process according to claim 1 and 2 characterized in that β-(meth)-alanine esters substituted at the nitrogen atom by hydroxy$C_1$-$C_6$-alkyl groups are used.

4. The process according to any one of claims 1 to 3 characterized in that N-(hydroxyalkyl)-substituted β-(meth)alanine-$C_1$-$C_6$-alkyl esters or -$C_1$-$C_6$-hydroxyalkyl esters are used.

5. The process according to any one of claims 1 to 4 characterized in that water-soluble polyamides of N-(hydroxyalkyl)β-(meth-)alanine esters are used.

6. The process according to any one of claims 1 to 4 characterized in that water-soluble polyamide ethers of N-(hydroxyalkyl)β-(meth-)alanine esters are used.

7. The process according to any one of claims 1 to 6 characterized in that at least 80 mol-% of the polymer are neutralized.

**Revendications**

**Revendications pour les Etats contractants suivants : AT, BE, CH, DE, DK, FR, GB, IT, LU, LI, MC, NL, PT, SE**

1. Agent d'absorption pulvérulent pour des liquides aqueux, à base d'un polymère pulvérulent, gonflable à l'eau, que l'on peut obtenir par la préparation et le chauffage d'un mélange, constitué de 100 parties en poids d'au moins un polymère contenant des radicaux carboxyle, gonflable à l'eau, qui contient au moins 0,2 équivalent de radicaux carboxyle par 100 parties en poids, qui se présentent pour 50 à 99% molaires sous forme neutralisée

   a) 0,001 à 5 parties en poids, de préférence 0,05 à 2 parties en poids, d'esters N-(hydroxyalkyl)β-(méth)-alaniniques et/ou
   b) 0,001 à 5 parties en poids, de préférence 0,05 à 2 parties en poids, de produits de polycondensation de a) dans
   c) 0,1 à 5 parties en poids d'eau et
   $d^1$) 0,2 à 10 parties en poids d'un diluant organique, miscible à l'eau, qui s'évapore au cours du chauffage du mélange et/ou
   $d^2$) 0,2 à 10 parties en poids d'un diluant organique, miscible à l'eau, qui ne s'évapore pas au cours du chauffage du mélange.

2. Agent d'absorption pulvérulent selon la revendication 1, caractérisé en ce que l'on obtient en chauffant le mélange selon la revendication 1 à une température de 100 à 250°C, de préférence 150 à 210°C.

3. Agent d'absorption pulvérulent selon les revendications 1 et 2, caractérisé en ce qu'on l'obtient par l'utilisation d'esters $\beta$-(méth)-alaniniques substitués par des radicaux hydroxyalkyle en $C_1$-$C_6$ sur l'atome d'azote.

4. Agent d'absorption pulvérulent suivant l'une quelconque des revendications 1 à 3, caractérisé en ce qu'on l'obtient par l'utilisation de $\beta$-(méth)alaninates d'alkyle en $C_1$-$C_6$ ou d'hydroxyalkyle en $C_1$-$C_6$ à substitution N-(hydroxyalkylique).

5. Agent d'absorption pulvérulent selon l'une quelconque des revendications 1 à 4, caractérisé en ce qu'on l'obtient par l'utilisation de polyamides solubles dans l'eau d'esters N-(hydroxyalkyl)$\beta$-(méth)alaniniques.

6. Agent d'absorption pulvérulent selon l'une quelconque des revendications 1 à 4, caractérisé en ce qu'on l'obtient par l'utilisation d'éthers polyamidiques solubles dans l'eau d'esters N-(hydroxyalkyl)$\beta$-(méth)alaniniques.

7. Agent d'absorption pulvérulent suivant l'une quelconque des revendications 1 à 6, caractérisé en ce qu'au moins 80% molaires du polymère sont neutralisés.

**Revendications pour l'Etat contractant suivant : GR**

1. Procédé de préparation d'un agent d'absorption pulvérulent pour des liquides aqueux, à base d'un polymère pulvérulent, gonflable à l'eau, caractérisé en ce que ceux-ci peuvent s'obtenir par la préparation et le chauffage d'un mélange, constitué de 100 parties en poids d'au moins un polymère contenant des radicaux carboxyle, gonflable à l'eau, qui contient au moins 0,2 équivalent de radicaux carboxyle par 100 parties en poids, qui se présentent pour 50 à 99% molaires sous forme neutralisée
a) 0,001 à 5 parties en poids, de préférence 0,05 à 2 parties en poids, d'esters N-(hydroxyalkyl)$\beta$-(méth)-alaniniques et/ou
b) 0,001 à 5 parties en poids, de préférence 0,05 à 2 parties en poids, de produits de polycondensation de a) dans
c) 0,1 à 5 parties en poids d'eau et
$d^1$) 0,2 à 10 parties en poids d'un diluant organique, miscible à l'eau, qui s'évapore au cours du chauffage du mélange et/ou
$d^2$) 0,2 à 10 parties en poids d'un diluant organique, miscible à l'eau, qui ne s'évapore pas au cours du chauffage du mélange.

2. Procédé suivant la revendication 1, caractérisé en ce que l'on chauffe le mélange obtenu suivant la revendication 1 à 100-250°C, de préférence 150 à 210°C.

3. Procédé suivant les revendications 1 et 2, caractérisé en ce que l'on utilise des esters $\beta$-(méth)-alaniniques substitués par des radicaux hydroxyalkyle en $C_1$-$C_6$ sur l'atome d'azote.

4. Procédé suivant l'une quelconque des revendications 1 à 3, caractérisé en ce que l'on utilise des $\beta$-(méth)alaninates d'alkyle en $C_1$-$C_6$ ou d'hydroxyalkyle en $C_1$-$C_6$ à substitution N-(hydroxyalkylique).

5. Procédé suivant l'une quelconque des revendications 1 à 4, caractérisé en ce que l'on utilise des polyamides solubles dans l'eau d'esters N-(hydroxyalkyl)$\beta$-(méth)alaniniques.

6. Procédé suivant l'une quelconque des revendications 1 à 4, caractérisé en ce que l'on utilise des éthers polyamidiques solubles dans l'eau d'esters N-(hydroxyalkyl)$\beta$-(méth)alaniniques.

7. Procédé suivant l'une quelconque des revendications 1 à 6, caractérisé en ce que l'on neutralise au moins 80% molaires du polymère.

**Revendications pour l'Etat contractant suivant : ES**

1. Procédé de préparation d'un agent d'absorption pulvérulent pour des liquides aqueux, à base d'un polymère pulvérulent, gonflable à l'eau, caractérisé en ce que ceux-ci peuvent s'obtenir par la préparation et le chauffage d'un mélange, constitué de 100 parties en poids d'au moins un polymère contenant des radicaux carboxyle, gonflable à l'eau, qui contient au moins 0,2 équivalent de radicaux carboxyle par 100 parties en poids, qui se présentent pour 50 à 99% molaires sous forme neutralisée

    a) 0,001 à 5 parties en poids, de préférence 0,05 à 2 parties en poids, d'esters N-(hydroxyalkyl)β-(méth)-alaniniques et/ou

    b) 0,001 à 5 parties en poids, de préférence 0,05 à 2 parties en poids, de produits de polycondensation de a) dans

    c) 0,1 à 5 parties en poids d'eau et

    d$^1$) 0,2 à 10 parties en poids d'un diluant organique, miscible à l'eau, qui s'évapore au cours du chauffage du mélange et/ou

    d$^2$) 0,2 à 10 parties en poids d'un diluant organique, miscible à l'eau, qui ne s'évapore pas au cours du chauffage du mélange.

2. Procédé suivant la revendication 1, caractérisé en ce que l'on chauffe le mélange obtenu suivant la revendication 1 à 100-250°C, de préférence 150 à 210°C.

3. Procédé suivant les revendications 1 et 2, caractérisé en ce que l'on utilise des esters β-(méth)-alaniniques substitués par des radicaux hydroxyalkyle en $C_1$-$C_6$ sur l'atome d'azote.

4. Procédé suivant l'une quelconque des revendications 1 à 3, caractérisé en ce que l'on utilise des β-(méth)alaninates d'alkyle en $C_1$-$C_6$ ou d'hydroxyalkyle en $C_1$-$C_6$ à substitution N-(hydroxyalkylique).

5. Procédé suivant l'une quelconque des revendications 1 à 4, caractérisé en ce que l'on utilise des polyamides solubles dans l'eau d'esters N-(hydroxyalkyl)β-(méth)alaniniques.

6. Procédé suivant l'une quelconque des revendications 1 à 4, caractérisé en ce que l'on utilise des éthers polyamidiques solubles dans l'eau d'esters N-(hydroxyalkyl)β-(méth)alaniniques.

7. Procédé suivant l'une quelconque des revendications 1 à 6, caractérisé en ce que l'on neutralise au moins 80% molaires du polymère.